(19) Europäisches Patentamt
European Patent Office
Office européen des brevets

(11) Veröffentlichungsnummer: **0 281 929**
**B1**

(12) **EUROPÄISCHE PATENTSCHRIFT**

(45) Veröffentlichungstag der Patentschrift: **27.12.90**

(51) Int. Cl.[5]: **C07B 35/06,** C07C 1/30

(21) Anmeldenummer: **88103227.0**

(22) Anmeldetag: **03.03.88**

(54) **Herstellung von Olefinen aus tertiären Alkylhalogeniden.**

(30) Priorität: **12.03.87 DE 3707903**

(43) Veröffentlichungstag der Anmeldung: **14.09.88 Patentblatt 88/37**

(45) Bekanntmachung des Hinweises auf die Patenterteilung: **27.12.90 Patentblatt 90/52**

(84) Benannte Vertragsstaaten: **BE CH DE FR GB IT LI NL**

(56) Entgegenhaltungen:
**DE-A-1 568 073**
**GB-A-17 234**
**US-A-3 341 615**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen(DE)**

(72) Erfinder: **Fischer, Martin, Dr., Elbinger Weg 1, D-6700 Ludwigshafen(DE)**

**EP 0 281 929 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Diese Erfindung betrifft ein neues Verfahren zur Herstellung von Olefinen durch Abspaltung von Halogenwasserstoff aus tertiären Alkylhalogeniden.

Bekanntlich lassen sich tertiäre Alkylhalogene durch Halogenwasserstoffabspaltung in Olefine überführen. Für diese Reaktion stehen verschiedene Methoden zur Verfügung. So kann man die Abspaltung von Halogenwasserstoff z.B. dadurch erreichen, daß man die tertiären Alkylhalogenide mit basischen Reagentien behandelt oder daß man sie einer Pyrolyse oder Solvolyse unterwirft (s. Houben-Weyl, Methoden der organischen Chemie, Band V/lb (1972, S. 134–135).

Bei der Halogenwasserstoffabspaltung mit basischen Reagentien, wie Alkalialkoholaten oder tertiären Aminen, werden stöchiometrische Mengen der Basen verbraucht. Bei der Solvolyse wird der abgespaltene Halogenwasserstoff nicht unter Verbrauch eines teuren Reaktionspartners chemisch gebunden. Dieser Vorteil wird jedoch dadurch aufgehoben, daß die Ausbeute an Olefin aufgrund konkurrierender Substitutionsreaktionen unbefriedigend und deutlich schlechter ist als bei der Basenbehandlung, die im Gegensatz zur unimolekularen Solvolyse nach einem bimolekularen Mechanismus abläuft (S. Patai, The Chemistry of Alkenes, 1964, S. 181). Solvolysen von tertiären Alkylhalogeniden werden üblicherweise in Mischungen von organischen Lösungsmitteln wie Ethanol, Dioxan oder Aceton mit Wasser durchgeführt. Am häufigsten wird Ethanol/Wasser im Verhältnis 4 : 1 eingesetzt (J. Amer. Chem. Soc. 1953, 75, 10–14).

In der GB-A 17 234 wird ein Verfahren zur Herstellung von ungesättigten Kohlenwasserstoffen beschrieben, bei dem man Kohlenwasserstoffe mit einem Halogenatom mit Wasserdampf behandelt und Kohlenwasserstoffe mit zwei Halogenatomen mit Wasser erhitzt. Bei diesem Verfahren sind Selektivität und Ausbeute gering. Aus der DE-A 1 568 073 ist bekannt, daß α-Chlorisopropylbenzole durch Erhitzen mit Wasser auf 50 bis 150°C unter Chlorwasserstoffabspaltung in Isopropenylbenzole übergehen. Es wird darauf hingewiesen, daß verwandte Verbindungen, wie tert.-Butylchlorid auf diese Weise nicht glatt in die entsprechenden Olefine übergeführt werden können.

Es wurde nun gefunden, daß man bei der Herstellung von Olefinen der Formeln I und II

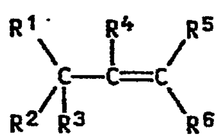

I

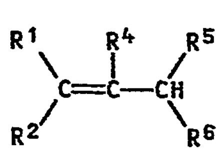

II

in denen $R^1$, $R^2$, $R^3$, $R^5$ und $R^6$ Wasserstoffatome oder Alkylgruppen mit 1 bis 6 C-Atomen bedeuten und $R^4$ für eine Alkylgruppe mit 1 bis 6 C-Atomen steht, durch Abspaltung von Halogenwasserstoff aus tertiären Alkylhalogeniden der Formel

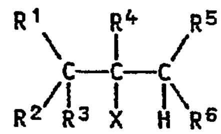

III,

in der $R^1$ bis $R^6$ die obengenannte Bedeutung haben und X für ein Chlor- oder Bromatom steht, wobei das Molgewicht der Alkylhalogenide 280 nicht übersteigt, besonders gute Ausbeuten erhält, wenn man die Halogenwasserstoffabspaltung durch Erhitzen der Alkylhalogenide in Nasser auf 40 bis 130°C vornimmt, wobei man die dabei gebildeten Olefine laufend aus dem Reaktionsgemisch abdestilliert.

Dieses vorteilhafte Ergebnis ist überraschend, da die Olefinausbeute bei der bekannten Solvolyse nach den Angaben in Houben-Weyl, Methoden der organischen Chemie, Band V/1b (1972), S. 170, in dem Maße abnimmt, in dem man den Wassergehalt im alkoholischen Reaktionsmilieu erhöht.

Die tertiären Alkylhalogenide der Formel III enthalten als Alkylgruppen solche mit 1 bis 6, vorzugsweise 1 bis 4 C-Atomen, wie Ethyl, Propyl, Isopropyl, Butyl oder Isobutylgruppen.

Im einzelnen seien die folgenden Alkylhalogenide genannt:

tert.-Amylchlorid, tert.-Amylbromid, tert.-Butylchlorid.

Man führt das erfindungsgemäße Verfahren in einem wäßrigen Medium durch. Beispielsweise setzt man das Alkylhalogenid der Formel III, das in Wasser nicht löslich ist, mit Wasser im Gewichtsverhältnis von 0,2 bis 2,5 zu 1 ein. Während des Erhitzens destilliert man das Olefin im Maße seiner Bildung aus dem wäßrigen Reaktionsgemisch ab. Je nach Siedepunkt des bei der Halogenwasserstoffabspaltung gebildeten Olefins oder Olefingemisches destilliert bei der Durchführung des Verfahrens ein mehr oder weniger großer Teil des Wassers als Azeotrop mit über. Das abdestillierte Wasser kann von den überdestillierten Olefinen durch Phasentrennung leicht abgetrennt werden.

Der aus dem Alkylhalogenid abgespaltene Halogenwasserstoff löst sich in dem im Reaktionsgefäß befindlichen Wasser. Wenn eine Säurekonzentration von 20 Gew.-% erreicht ist, wird die Olefinbildung merklich langsamer. Zweckmäßigerweise bricht man die Reaktion ab, wenn im Reaktionsgemisch die Sättigungskonzentration an Säure erreicht ist. Sollte das Alkylhalogenid dann nicht vollständig umgesetzt sein, so kann die Halogenwasserstoffabspaltung durch Austausch der wäßrigen Säure gegen Wasser wieder neu in Gang gebracht werden.

Um zu vermeiden, daß ein Teil des Alkylhalogenids mit dem (oder den) entstandenen Olefin(en) überdestilliert, ist es zweckmäßig, zwischen Reaktionsgefäß und Kühler eine Kolonne z.B. mit 2 bis 6 Trennstufen anzubringen, die nicht umgesetztes Alkylhalogenid zurückhält.

Die Reaktionstemperatur richtet sich nach den Siedepunkten des eingesetzten Alkylhalogenids und des abdestillierenden Olefin(gemische)s. Sie wird zweckmäßigerweise auf 10 bis 60°C oberhalb des Siedepunktes der entstehenden Olefine bzw. von deren Azeotroptemperatur mit Wasser eingestellt.

Man kann die Reaktion im Druckbereich von 0,3 bar bis 5 bar durchführen. Den geringsten technischen Aufwand erfordert die Durchführung bei Normaldruck oder bei geringfügig erhöhtem oder vermindertem Druck. Das Verfahren der Erfindung kann kontinuierlich oder ansatzweise durchgeführt werden. Nach einer vorteilhaften Ausführungsform der Erfindung verfährt man so, daß man das Wasser, das man im Reaktionsgefäß vorlegt, auf die gewünschte Temperatur bringt und das Alkylhalogenid in dem Maße zudosiert, in dem das (oder die) gebildete(n) Olefin(e) abdestilliert (bzw. abdestillieren). Diskontinuierliche Ansätze benötigen in der Regel Reaktionszeiten von 1 bis ca. 12 Stunden. Längere Reaktionszeiten können ohne Beeinträchtigung der Ausbeute toleriert werden, wenn z.B. das Olefin nicht schnell genug abdestilliert werden kann.

Bei dem Verfahren der Erfindung nimmt man die Halogenwasserstoffabspaltung in Wasser vor. Die Zugabe von Lösungsmitteln ist nicht erforderlich aber möglich. Da während der Umsetzung Halogenwasserstoff frei wird, wodurch in dem wäßrigen Reaktionsmedium die entsprechende Säure gebildet wird, kann man auch von einer verdünnten wäßrigen Halogenwasserstoffsäure ausgehen. Als wäßrige Reaktionsmedien kommen somit neben Wasser auch wäßrige Gemische mit Halogenwasserstoffen oder Lösungsmitteln in Betracht.

Das eingesetzte Wasser kann z.B. 0 bis 70, vorzugsweise 0 bis 40, insbesondere 0 bis 10 Gew.-% an Lösungsmitteln und 0 bis 15, vorzugsweise 0 bis 5 Gew.-% an Halogenwasserstoff enthalten. Als Lösungsmittel kommen solche in Betracht, die unter den Verfahrensbedingungen gegenüber Halogenwasserstoffsäuren beständig sind. Beispielsweise seien hier Toluol, Chloroform, Dichlorethan und Methylenchlorid genannt.

Beispiel 1

500 ml Wasser werden in einem Rührkolben auf 65 bis 70°C erhitzt. Im Verlauf von 6 Std. tropft man 586 g tert.-Amylchlorid zu. Das entstehende Olefingemisch wird dabei fortlaufend über eine 30 cm lange mit 5 mm-Glasringen gefüllte Kolonne abdestilliert, wobei eine Kopftemperatur von 38°C nicht überschritten werden soll. Es destillieren 14 ml Wasser und 354 g eines Olefingemisches über, das aus 90 % 2-Methylbuten-2 und 10 % 2-Methylbuten-1 besteht.

Beispiel 2

300 ml Wasser werden in einem mit aufgesetztem Rückflußkühler und angeschlossener Kühlfalle versehenen Rührkolben auf 50 bis 55°C erwärmt. Man läßt 50 g tert.-Butylchlorid zutropfen und hält das Gemisch unter Rückfluß am Sieden. Nach einer Stunde beginnt sich in der auf -70°C gekühlten Kühlfalle Isobutylen abzuscheiden. Die Induktionsphase bis zum Beginn der Olefinbildung kann durch Zusatz von 10 ml konz. Salzsäure ausgeschaltet werden. In 6 Std. läßt man weitere 100 g tert.-Butylchlorid zutropfen. Insgesamt sammeln sich in der Kühlfalle 82 g Isobutylen an.

**Patentansprüche**

1. Verfahren zur Herstellung von Olefinen der Formeln I und II

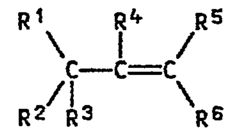

I

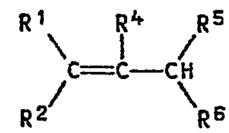

II

in denen $R^1$, $R^2$, $R^3$, $R^5$ und $R^6$ Wasserstoffatome oder Alkylgruppen mit 1 bis 6 C-Atomen bedeuten und $R^4$ für eine Alkylgruppe mit 1 bis 6 C-Atomen steht, durch Abspaltung von Halogenwasserstoff aus tertiären Alkylhalogeniden der Formel

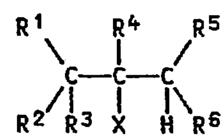

III,

in der $R^1$ bis $R^6$ die obengenannte Bedeutung haben und X für ein Chlor- oder Bromatom steht, wobei das Molgewicht der Alkylhalogenide 280 nicht übersteigt, dadurch gekennzeichnet, daß man die Halogenwasserstoffabspaltung durch Erhitzen der Alkylhalogenide in Wasser auf 40 bis 130°C vornimmt, wobei man die dabei gebildeten Olefine laufend aus dem Reaktionsgemisch abdestilliert.

2. Verfahren nach Anspruch 1, dadurch gekennzeichnet, daß man 0,2 bis 2,5 Gewichtsteile Alkylhalogenid auf 1 Gewichtsteil Wasser einsetzt.

3. Verfahren nach Anspruch 1, dadurch gekennzeichnet, daß man die Umsetzung bei 0,3 bis 5 bar durchführt.

4. Verfahren nach Anspruch 1, dadurch gekennzeichnet, dass man als Alkylhalogenid tert.-Amylchlorid oder tert.-Butylchlorid verwendet.

## Claims

1. A process for the preparation of an olefin of the formulae I or II,

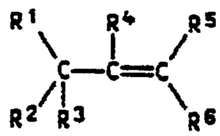

I

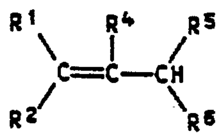

II

where $R^1$, $R^2$, $R^3$, $R^5$ and $R^6$ are hydrogen or alkyl having from 1 to 6 carbon atoms, and $R^4$ is alkyl having from 1 to 6 carbon atoms, by eliminating hydrogen halide from a tertiary alkyl halide of the formula,

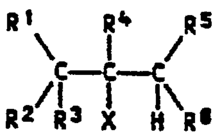

III

where $R^1$ to $R^6$ are as defined above, and X is chlorine or bromine, where the molecular weight of the alkyl halide does not exceed 280, which comprises eliminating the hydrogen halide by heating the alkyl halide to from 40 to 130°C in water and removing the resultant olefin continuously from the reaction mixture by distillation.

2. A process as claimed in claim 1, wherein from 0.2 to 2.5 parts by weight of alkyl halide are employed per part by weight of water.

3. A process as claimed in claim 1, wherein the reaction is carried out at from 0.3 to 5 bar.

4. A process as claimed in claim 1, wherein the alkyl halide used is tert-amyl chloride or tert-butyl chloride.

## Revendications

1. Procédé de préparation d'oléfines de formules I et II

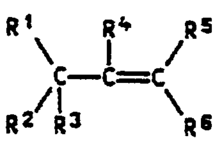

I

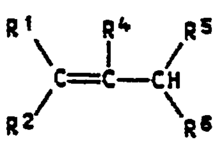

II

dans lesquelles $R^1$, $R^2$, $R^3$, $R^5$ et $R^6$ sont des atomes d'hydrogène ou des groupements alkyle ayant de 1 à 6 atomes de carbone et $R^4$ est mis pour un groupement alkyle ayant de 1 à 6 atomes de carbone, par élimination d'acide halohydrique à partir d'halogénures d'alkyle tertiaire de formule

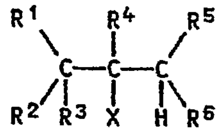

III,

dans laquelle $R^1$ à $R^6$ ont les définitions données ci-dessus et X est mis pour un atome de chlore ou de brome, la masse moléculaire de l'halogénure d'alkyle ne dépassant pas 380, caractérisé en ce qu'on effectue l'élimination de l'acide halohydrique par chauffage de l'halogénure d'alkyle dans l'eau à 40–130°C, en enlevant par distillation en continu du mélange réactionnel les oléfines ainsi formées.

2. Procédé selon la revendication 1, caractérisé en ce qu'on utilise 0,2 à 2,5 parties en poids d'eau pour 1 partie en poids d'eau.

3. Procédé selon la revendication 1, caractérisé en ce qu'on mène la réaction à 0,3–5 bar.

4. Procédé selon la revendication 1, caractérisé en ce qu'on utilise comme halogénure d'alkyle le chlorure de tert.-amyle ou le chlorure de test-butyle.